# EUROPEAN PATENT APPLICATION

(11) **EP 4 681 705 A1**
(43) Date of publication of application: **21.01.2026**
(21) Application number: 24770052.9
(22) Date of filing: 26.04.2024
(51) Int. Cl.: A61K 9/70, A61K 31/135, A61K 47/32, A61K 47/14, A61K 47/12, A61P 25/16

(54) **TRANSDERMAL PATCH COMPRISING RANSAGILINE MESYLATE AND PREPARATION METHOD THEREFOR**

(30) Priority: 16.03.2023 CN 202310254976; 18.09.2023 CN 202311201304
(71) Applicant: Shanghai World Leader Pharmaceutical Co., Ltd., Shanghai 201499 (CN)
(72) Inventor: GONG, Jinkang, Shanghai 201499 (CN); XU, Zhihui, Shanghai 201499 (CN); SUN, Changlei, Shanghai 201499 (CN)
(74) Representative: Ponti & Partners, S.L.P
(86) International application number: PCT/CN2024/089978
(87) International publication number: WO 2024/188365

(57) **Abstract**

A transdermal patch comprising ransagiline mesylate and a preparation method therefor. A hydrogen bond complex is formed by means of the combined action of ransagiline mesylate, crospovidone and a crystallization inhibitor, so that ransagiline mesylate is present in a drug carrier in an amorphous state, thereby avoiding an unstable form of a free base. The transdermal patch further comprises a release-promoting agent selected from one or more of levulinic acid, octanoic acid and undecylenic acid.

## Description

### TECHNICAL FIELD

The present disclosure relates to the field of pharmaceutical preparation transdermal administration, and in particular, to a transdermal patch containing rasagiline mesylate and a preparation method therefor.

### BACKGROUND ART

Rasagiline is an irreversible selective inhibitor of monoamine oxidase-B (MAO-B), which is used for the treatment of degenerative Parkinson's disease (PD) of central nervous system. This pharmaceutical oral preparation has gone on sale in Europe, with two specifications of 0.5 mg/d and 1 mg/d. Rasagiline is an irreversible inhibitor of monoamine oxidase, with both strong therapeutic efficacy and high safety, and no serious adverse reaction is found after oral administration at 20 mg/d for 2 weeks. Due to on-off effect in patients with Parkinson's disease, when they are in an off-stage, movement of patients with Parkinson's disease is limited, and it is inconvenient for the patients themselves to move, resulting in difficulty in oral administration. Transdermal administration can significantly improve compliance of the patients. Meanwhile, oral preparations have relatively low bioavailability of only 36%, while transdermal administration delivers drugs directly into blood circulation, and significantly improves bioavailability of the drugs. Currently, in the Parkinson's disease treatment, Rotigotine transdermal patch has been marketed. Compared with oral drugs, the Rotigotine transdermal patch has a clinical advantage of improving patient compliance. Development of rasagiline transdermal patch can facilitate the administration of drugs to patients with Parkinson's disease, and improve patient compliance and bioavailability of the drugs. Data have shown that under weakly alkaline conditions, no matter rasagiline free base is carried in polyacrylate pressure-sensitive adhesives, silicone pressure-sensitive adhesives or polyvinyl alcohol, a main drug content declines rapidly, even with complete degradation, and is highly unstable. However, as mesylate drug of rasagiline, no matter rasagiline mesylate is carried in polyacrylate pressure-sensitive adhesives, silicone pressure-sensitive adhesives, or polyvinyl alcohol, a main drug content has no change. Therefore, rasagiline mesylate is quite suitable for a transdermal patch form.

Transdermal drug delivery system (TDDS) or transdermal therapeutic system (TTS) is a method for absorption of drugs through the skin. Drugs are absorbed through the skin into the human blood circulation, and reach an effective blood concentration, to implement a new administration route for treating or preventing diseases. There are mainly four advantages: (1) avoiding gastrointestinal tract irritation and drug decomposition, meanwhile avoiding a liver first-pass effect, and improving bioavailability of drugs; (2) stable and long-lasting release rate, and reducing toxic and side effects caused by fluctuation of peaks and valleys; (3) no need of frequent administration, and improving patient compliance (the elderly, infants, and critical patients); and (4) convenient and flexible administration, and timely interruption of administration when toxic and side effects are found.

To sum up, formulating rasagiline mesylate as a transdermal patch for administration through the skin can have many advantages and provide more choices for patients.

Currently, rasagiline has several modes of transdermal administration as follows.
(1) Spray percutaneous absorption (US2004013620), characterized by use of a characteristically structured dermal penetration enhancer, but with suboptimal transdermal absorption efficacy.
(2) Transdermal patch, where although a method in patent CN101032474B, Rasagiline Transdermal Patch for Treatment or Prophylaxis of Nervous System Disease and Preparation Process therefor can obtain good transdermal permeation effect, drug stability is not good. In this patent, pH is relatively high, rasagiline mainly exists in the form of free base, and an R-NH-C≡ CH bond between a secondary amine group thereof and a linked alkynyl group is liable to be broken, thereby causing degradation; therefore, rasagiline is difficult to remain stable in an alkaline matrix environment for a long time, particularly when in the free base, the instability is more obvious, and the drug stability is not good, which is not conducive to long-term storage.

Rasagiline has many advantages through transdermal administration. Existing transdermal administration techniques of rasagiline have defects: (1) transdermal absorption effect of the spray percutaneous absorption (US2004013620) is not ideal; and (2) the transdermal patch system has a relatively high pH, rasagiline mainly exists in the form of free base, and when rasagiline is in the state of free base, it is quite unstable, and it is difficult to achieve long-term storage.

### SUMMARY

The present disclosure primarily provides a transdermal patch containing rasagiline mesylate, aiming at solving problems of existing transdermal patches, such as unideal absorption efficacy, instability, easy drug precipitation and difficulty in long-term storage. The present disclosure designs a technical solution of carrying rasagiline mesylate in a water-insoluble carrier. Firstly, a transdermal patch containing rasagiline mesylate is provided, containing a backing layer, a matrix layer and a protective layer.

Solution 1 is characterized in that the matrix layer (called as a matrix mixture when not coated in the present application for patent) contains following components in mass percentage: 0.5-8.0% of rasagiline mesylate, 40-80% of a binder, 3-25% of crospovidone, 1-5% of a crystallization inhibitor, 1-15% of a penetration enhancer, 1-10% of a plasticizer, and 0.001-0.5% of an antioxidant, with a mass ratio of rasagiline mesylate to crospovidone being 1:5 to 1:15, and a content of the binder being finally adjusted to formulate a mass percentage of 100%. Preferably, rasagiline mesylate is 0.5-5.0%.

Solution 2 is characterized in that the matrix layer (called as a matrix mixture when not coated in the present application for patent) contains following components in mass percentage: 0.5-5.0% of rasagiline mesylate, 40-80% of a binder, 3-25% of crospovidone, 0.1-5.0% of a release promoter, 1-15% of a penetration enhancer, 0.5-10% of a plasticizer, and 0.001-0.5% of an antioxidant, with a mass ratio of rasagiline mesylate to crospovidone being 1:5 to 1:15, and a content of the binder being finally adjusted to formulate a mass percentage of 100%.

The above technical solutions have following advantageous effects.

Currently, binders selected for use in TDDS drug delivery systems are mainly binders such as acrylates, organosiloxanes, and hot melt adhesives. Several binders mentioned in the above are all organic solvent dissolving systems, mainly used in transdermal patches of liposoluble drugs. Rasagiline mesylate and solvent-based binders have the problem of incompatibility. The present disclosure innovatively employs the drug carrier crospovidone conventionally used for carrying liposoluble or water-insoluble drugs, to carry the water-soluble drug rasagiline mesylate. Hydrogen bond complexation is formed by a combined action of rasagiline mesylate, the drug carrier and the crystallization inhibitor, so that rasagiline mesylate is stably present in the drug carrier in an amorphous state, and no crystallization and precipitation occur to the prepared patch. Furthermore, rasagiline exists in the form of salt, avoiding the instability of a preparation. Such a combination form is disclosed in transdermal patches for the first time.

In particular, the inventors have found by exploration and experiments that in the case of a low drug loading (<0.5%), in formulations in the absence of PVPP, rasagiline mesylate will not crystallize and precipitate heavily, since the system has some solubility for the drug. However, when the drug content is higher than 0.50%, significant crystallization and precipitation of rasagiline mesylate will occur. Given the druggability of transdermal patch drugs, even minimal crystallization may affect the dissolution, release, and permeation of the drugs to some extent, thereby influencing the drug efficacy. Therefore, a low drug loading and a certain proportion of crystallization will lead to difficulty in preparation of drugs, and it is necessary to increase the drug loading and solve the crystallization problem, while addition of PVPP can increase the drug loading without crystallization and precipitation. It has been found by exploration and experiments that the maximum drug loading of the system is no more than 5.0% by mass of rasagiline mesylate. If the drug loading is too high, it will be difficult to fulfill the preparation process. Furthermore, it is found through experiments that when the mass ratio of rasagiline mesylate to crospovidone is 1:5 to 1:15, the system has relatively good druggability.

Therefore, patches with a high drug loading could be prepared based on the formulation of the transdermal patch containing rasagiline mesylate provided by the present disclosure, without precipitation during long-term storage, and meets druggability. Upon testing, in the case where the thickness of the matrix layer of the finally prepared patch is appropriate, the effective drug contained in unit area is 0.058-0.565 mg/cm² calculated on the basis of rasagiline free base, achieving the drug loading of the salt drug in a relatively high concentration in the organic solvent pressure-sensitive adhesive system without crystallization and precipitation.

Further, the formulation of the present disclosure does not employs inorganic salts, inorganic bases or organic bases, thereby overcoming problems such as skin discomfort and redness which are necessarily caused by applying the patch to outer skin of patients by adjusting pH value using the inorganic salts, inorganic bases or organic bases in the technical solution of CN101032474B (although CN101032474B emphasizes adjusting pH to an appropriate range, the addition of inorganic salts, inorganic bases or organic bases will inevitably stimulates the skin along with discomfort).

The drug of the present disclosure can be accurately, stably and durably released and delivered, in the absence of a controlled-release film.

A transdermal effect is ideal, and a cumulative permeation quantity of 24 h can be up to 20-100 µg/cm².

Use experience is excellent, and the drug carrier is insoluble in water and ethanol, and exists in a granular state in the preparation, which can avoid problems of sticky feel and binder residual caused by introducing a high proportion of a high molecular crystallization inhibitor, which affects comfort of use.

In solutions 1 and 2,
Preferably, the binder is preferably acrylate pressure-sensitive adhesive.

Preferably, the acrylate pressure-sensitive adhesive is selected from one or a combination of several of DURO-TAK 387-2516, DURO-TAK 387-2052, DOR0-TAK 87-4098 and DURO-TAK 387-2287.

Preferably, the crystallization inhibitor is at least one substance selected from the group consisting of: povidone K90, povidone K30, povidone K25, povidone K17, povidone K12, and copovidone VA 64.

Preferably, the crystallization inhibitor is selected from one or a combination of several of povidone K25, povidone K17, povidone K12 and copovidone VA 64.

Preferably, the penetration enhancer is selected from at least one of following substances: propylene glycol monocaprylate, polyglyceryl fatty acid ester, glyceryl monooleate, glyceryl monolinoleate, glyceryl monostearate, Span 80 and Span 60.

Preferably, the penetration enhancer is selected from propylene glycol monocaprylate or polyglyceryl fatty acid ester.

Preferably, the plasticizer is selected from one or a combination of several of triethyl citrate, glyceryl triacetate and dibutyl phthalate.

Preferably, the plasticizer is selected from preferably triethyl citrate.

Preferably, the antioxidant is selected from one or more of vitamin E (tocopherol), tocopheryl acetate, tocopheryl succinate, tocopheryl nicotinate, tocopheryl palmitate, tocopheryl linoleate and tocopheryl phosphate.

Preferably, the antioxidant is selected from vitamin E (tocopherol).

Preferably, the backing layer is selected from one of SCOTCHPAK^{™}9738, SCOTCHPAK^{™}9730, and SCOTCHPAK^{™}1109.

Preferably, the protective layer is selected from one of SCOTCHPAK^{™}1022 or SCOTCHPAK^{™}2504.

In solution 1,
Preferably, the matrix layer further contains following component in mass percentage: 0.1-5.0% of a release promoter.

Preferably, a mass ratio of rasagiline mesylate to the crystallization inhibitor is 3:1-3:5.

Preferably, a mass ratio of the plasticizer to the penetration enhancer is 1:1-1:5.

In solution 2,
Preferably, the matrix layer further contains following component in mass percentage: 1-5% of a crystallization inhibitor.

Preferably, a mass ratio of the release promoter to rasagiline mesylate is 1:1-1:4.

Preferably, a mass ratio of the plasticizer to the penetration enhancer is 1:1-1:10.

Preferably, the mass ratio of the release promoter to the penetration enhancer is 1:1-1:15.

In solutions 1 and 2,
The release promoter is selected from one or a combination of several of levulinic acid, caprylic acid, and undecylenic acid, preferably levulinic acid.

Preferably, the backing layer is selected from one of SCOTCHPAK^{™}9738, SCOTCHPAK^{™}9730, and SCOTCHPAK^{™}1109; and the protective layer is selected from one of SCOTCHPAK^{™}1022, SCOTCHPAK^{™}2504, and 75E-0010BD.

In method 1,
The present disclosure further provides a preparation method for a transdermal patch containing rasagiline mesylate, including:
step 1: preparing a drug-containing adhesive layer (a matrix layer, called as a matrix mixture when not coated in the present application for patent), wherein 0.5-8% of rasagiline mesylate (preferably 0.5-5.0%), 1-5% of a crystallization inhibitor, 1-15% of a penetration enhancer, 1-10% of a plasticizer, and 0.001-0.5% of an antioxidant are weighed, dissolved in a solvent, and mixed and stirred until all solids are completely dissolved to provide a homogeneous solution; 3-25% of crospovidone is added into the homogeneous solution, followed by pre-mixing and swelling sufficiently and then homogenizing, to provide a white emulsion liquid; and 40-80% of a binder is added to the white emulsion liquid, followed by mixing and stirring uniformly to provide an intermediate solution;
step 2: coating and drying to remove the solvent, wherein the intermediate solution is coated on a release film (protective layer), with a wet film thickness of 100-500 µm, and then dried to provide a dried matrix, with a dried film thickness of 20-250 µm; and
step 3: laminating, compounding and cutting, wherein the backing film (backing layer) is laminated and compounded with the dried matrix, and then cut into patches.

In step 1, a mass ratio of rasagiline mesylate to crospovidone is 1:5 to 1:15, and a content of the binder is finally adjusted to formulate a mass percentage of 100%.

In method 2,
The present disclosure further provides a preparation method for a transdermal patch containing rasagiline mesylate, including:
step 1: preparing a drug-containing matrix layer (called as a matrix mixture when not coated in the present application for patent), wherein 0.5-5.0% of rasagiline mesylate, 0.1-5.0% of a release promoter, 1-15% of a penetration enhancer, 0.5-10% of a plasticizer, and 0.001-0.5% of an antioxidant are weighed, dissolved in a solvent, and mixed and stirred until all solids are completely dissolved to provide a homogeneous solution; 3-25% of crospovidone is added into the homogeneous solution, followed by pre-mixing and swelling sufficiently and then homogenizing, to provide a white emulsion liquid; and 40-80% of a binder is added to the white emulsion liquid, followed by mixing and stirring uniformly to provide an intermediate solution;
step 2: coating and drying to remove the solvent, wherein the intermediate solution is coated on a protective layer, with a wet film thickness of 100-500 µm, and then dried to provide a dried matrix, with a dried film thickness of 20-250 µm; and
step 3: laminating, compounding and cutting, wherein the backing layer is laminated and compounded with the dried matrix, and then cut into patches.

In step 1, a mass ratio of rasagiline mesylate to crospovidone is 1:5 to 1:15, and a content of the binder is finally adjusted to formulate a mass percentage of 100%.

The above technical solutions have following beneficial effects.

Hydrogen bond complexation is formed by means of a combined action of rasagiline mesylate, the drug carrier crospovidone, and the crystallization inhibitor, so that rasagiline mesylate is present in the drug carrier in an amorphous state, the prepared patch has no crystallization phenomenon, and rasagiline exists in the form of salt, avoiding instability of the preparation.

In methods 1 and 2,
Preferably, the solvent in step 1 is selected from one or a combination of two of anhydrous ethanol or isopropyl alcohol, preferably anhydrous ethanol.

Preferably, the solvent in step 1 is selected from anhydrous ethanol.

Preferably, drying in step 2 is carried out by a three-stage drying method, with drying at 20-30 °C for 2-10 min in a first stage, preferably drying at 20-25 °C for 2-10 min, drying at 45-65 °C for 2-10 min in a second stage, and drying at 65-85 °C for 2-10 min in a third stage.

In method 1,
Preferably, the matrix layer further contains following component in mass percentage: 0.1-5.0% of a release promoter.

Preferably, a mass ratio of rasagiline mesylate to the crystallization inhibitor is 3:1-3:5.

Preferably, a mass ratio of the plasticizer to the penetration enhancer is 1:1-1:5.

In method 2,
the matrix layer further contains following component in mass percentage: 1-5% of a crystallization inhibitor.

Preferably, a mass ratio of the release promoter to rasagiline mesylate is 1:1-1:4.

Preferably, a mass ratio of the plasticizer to the penetration enhancer is 1:1-1:10.

Preferably, a mass ratio of the release promoter to the penetration enhancer is 1:1-1:15.

In methods 1 and 2, preferably, an application area after the cutting is 3 cm²-30 cm².

In particular, unless otherwise specified, numerical ranges of in the text of the present disclosure have an error of ±10%, since measurement results of measuring instrument are inevitably erroneous.

### BRIEF DESCRIPTION OF DRAWINGS

FIG. 1 is a cross-sectional structural view of a transdermal patch of the present disclosure.
FIG. 2 shows Q-t relation curves of formulations of Examples 3-7 in the present disclosure.
FIG. 3 shows Q-t relation curves of formulations of Examples 8-9 in the present disclosure.
FIG. 4 shows photographs of patches prepared according to formulations of Examples 1'-3' in the present disclosure observed under a polarizing microscope after placement.

### DETAILED DESCRIPTION OF EMBODIMENTS

Preferred examples in following description are merely exemplary, and those skilled in the art could think of other obvious variants. Basic principle of the present disclosure defined in the following description is applicable to other embodiments, variations, modifications, equivalents and other technical solutions without departing from the spirit and scope of the present disclosure.

In the following description, [Examples 1-2] compared formulations with PVPP (crospovidone) and without PVPP, aiming at demonstrating that PVPP can carry a water-soluble drug rasagiline mesylate.

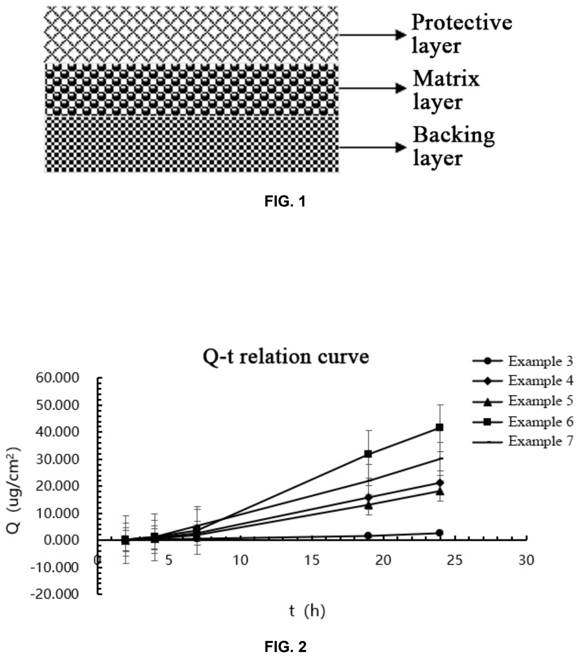

[Examples 3-7] screened formulation IVPT (IVPT: English abbreviation for *in vitro* permeation testing) without a penetration enhancer and with a penetration enhancer having HLB (HLB: abbreviation for hydrophile lipophilic balance) values of 1-5, to further demonstrate the necessity of using the penetration enhancer and the reason why the penetration enhancer with HLB values of 3-5 is preferred. IVPT results of [Examples 3-7] are shown in FIG. 2, and permeation quantity (Q)-time (t) equations are listed in Table 1.

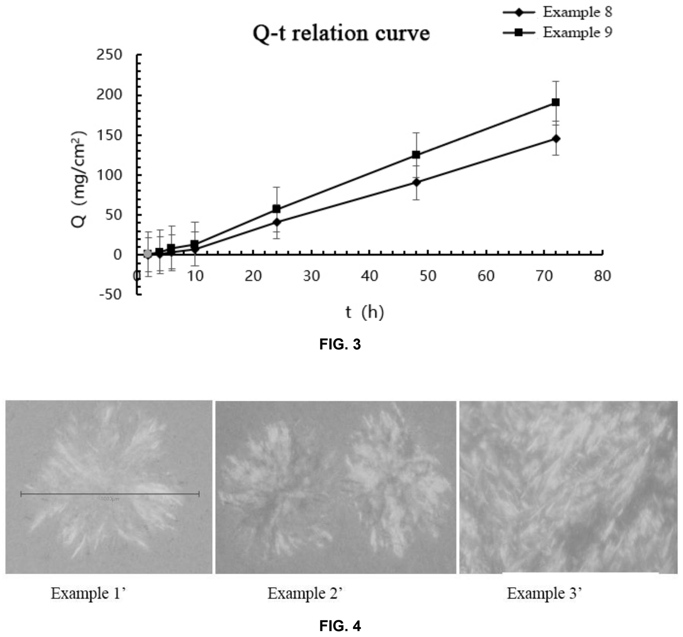

[Examples 8-9] further combined penetration enhancers with different HLB values of 3-5 (HLB: abbreviation for hydrophile lipophilic balance) and plasticizer, and performed optimization and screening through IVRT (English abbreviation for *in vitro* release testing) and IVPT (English abbreviation for *in vitro* permeation testing). IVPT results of [Examples 8-9] are shown in FIG. 3, and permeation quantity (Q)-time (t) equations are listed in Table 2.

In the present disclosure, research of *in vitro* release testing (IVRT) adopts method 4 (paddle over disk) of 0931 of the *Pharmacopoeia of the People's Republic of China* (2020 edition). Dissolution instrument and high performance liquid chromatography are utilized to calculate a cumulative release amount within a certain sampling time, and release rate (%)-time (t) relation curves of rasagiline are plotted. Implementation solution of IVRT is as follows:
Release medium: pH 4.5 acetate buffer (weighing sodium acetate trihydrate 2.99 g, adding water 1,000 mL for dissolution, and adjusting pH with acetic acid to 4.5); pH 7.4 phosphate buffer (weighing potassium dihydrogen phosphate 6.80 g and sodium hydroxide 1.564 g, and adding water 1,000 mL for dissolution);
Medium volume/temperature/rotational speed/sample amount: 900 mL, 32±0.5 °C, 50 r/min, 3 parts per batch;
Sampling time points: 0.5, 1, 2, 4, 8, 12, 24, 26, 48, 60, 72 h (generally 24 h);
Chromatography conditions are: chromatographic column: Agilent Zorbax SB-C18 4.6×250 mm, 5 µm; mobile phase: 10 mM KH2PO4: ACN=80:20; flow rate: 1.0 ml/min; column temperature: 40 °C; detection wavelength: 210 nm; injection volume: 50 µl; and collection time: 6 min.
Research of *in vitro* permeation testing (IVPT) utilized Franz cell (*in vitro* transdermal experimental system) and Bama miniature pig skin to assess permeability of rasagiline mesylate. The receiving solution was 0.01% gentamicin sulfate (pH 7.4 PBS). Contents of rasagiline at different sampling points in a receiving tank were determined using high performance liquid chromatography, a cumulative permeation quantity of rasagiline was calculated, and cumulative permeation quantity (Q)-time (t) relation curves were plotted.

In addition, in order to further demonstrate that the patch has relatively good use performance, transdermal patches prepared with formulation and process of [Example 9] were further tested for adhesive force and peel force.

Finally, in order to further demonstrate that the patch has good stability, the transdermal patches prepared with the formulation and process of [Example 9] were further tested for stability. Results are listed in Table 3.

### [Example 1]

| Ingredient | Formulation Ratio (%) |
|---|---|
| Rasagiline mesylate | 1.00 |
| DURO-TAK 387-2052 | 92.00 |
| Triethyl citrate | 2.00 |
| Propylene glycol monocaprylate | 4.00 |
| Polyglyceryl oleate | 1.00 |
| Total (100 g, the same below) | 100.00 |
| Solvent (anhydrous ethanol, in g, the same below) | 25.00 |

Step 1: preparing a drug-containing adhesive layer (matrix layer). (1) Rasagiline mesylate, triethyl citrate, propylene glycol monocaprylate, polyglyceryl oleate, and anhydrous ethanol were mixed and stirred until all solids were completely dissolved, so as to provide a transparent homogeneous solution; and (2) DURO-TAK 387-2052 was added into the above liquid, followed by mixing and stirring uniformly to provide an intermediate solution.

Step 2: coating and drying to remove solvent. The intermediate solution was coated on a SCOTCHPAK^{™}1022 release film (protective layer), with a wet film thickness of 300 µm, and then dried. Drying was carried out by a three-stage drying method, i.e., drying at 20 °C for 5 min, drying at 50 °C for 5 min, and drying at 70 °C for 10 min, to provide a dried matrix, with a thickness of 120 µm.

Step 3: laminating, compounding and cutting. A SCOTCHPAK^{TM9738} backing film (backing layer) was laminated and compounded with the above dried matrix.

After patches prepared by the above formulation and process were left at room temperature for 3 days, a lot of crystals were precipitated.

### [Example 2]

| Ingredient | Formulation Ratio (%) |
|---|---|
| Rasagiline mesylate | 1.00 |
| DURO-TAK 387-2052 | 82.00 |
| Crospovidone | 10.00 |
| Triethyl citrate | 2.00 |
| Propylene glycol monocaprylate | 4.00 |
| Polyglyceryl oleate | 1.00 |
| Total | 100.00 |
| Solvent (anhydrous ethanol) | 25.00 |

Step 1: preparing a drug-containing adhesive layer (matrix layer). (1) Rasagiline mesylate, triethyl citrate, propylene glycol monocaprylate, polyglyceryl oleate, and anhydrous ethanol were mixed and stirred until all solids were completely dissolved, so as to provide a transparent homogeneous solution; (2) crospovidone was added into the above solution, followed by pre-mixing and stirring uniformly and then homogenizing, to provide a white emulsion liquid; and (3) DURO-TAK 387-2052 was added into the above liquid, followed by mixing and stirring uniformly to provide an intermediate solution.

Step 2: coating and drying to remove solvent. The intermediate solution was coated on a SCOTCHPAK^{™}1022 release film (protective layer), with a wet film thickness of 300 µm, and then dried. Drying was carried out by a three-stage drying method, i.e., drying at 25 °C for 5 min, drying at 55 °C for 8 min, and drying at 75 °C for 10 min, to provide a dried matrix, with a thickness of 120 µm.

Step 3: laminating, compounding and cutting. A SCOTCHPAK^{TM}9738 backing film (backing layer) was laminated and compounded with the above dried matrix, and then cut into patches of 10 cm².

The patches prepared by the above formulation and process were left under four conditions of room temperature, high temperature (40 °C), refrigeration (4 °C), and freezing (-18 °C) for 60 days, and no crystal precipitation was observed. It can be seen therefrom that PVPP can carry the water-soluble drug rasagiline mesylate.

[Example 3-7] evaluated IVPT by preparing formulations without a penetration enhancer and with a penetration enhancer having HLB values of 1-5, to further demonstrate the necessity of the penetration enhancer and the reason for selecting the penetration enhancer with HLB values of 3-5. Permeation quantity (Q)-time (t) equations of [Examples 3-7] are listed in Table 1, and IVPT curves are shown in FIG. 2.

### [Example 3] Without Penetration Enhancer

| Ingredient | Formulation Ratio (%) |
|---|---|
| Rasagiline mesylate | 3.00 |
| DURO-TAK 387-2052 | 74.90 |
| Crospovidone | 15.00 |
| Povidone K25 | 2.00 |
| Vitamin E | 0.10 |
| Triethyl citrate | 5.00 |
| Total | 100.00 |
| Solvent (anhydrous ethanol) | 30.00 |

Step 1: preparing a drug-containing adhesive layer (matrix layer). (1) Rasagiline mesylate, vitamin E, triethyl citrate and anhydrous ethanol were mixed and stirred until all solids were completely dissolved, so as to provide a transparent homogeneous solution; (2) crospovidone was added into the above solution, followed by pre-mixing and stirring uniformly and then homogenizing, to provide a white emulsion liquid; and (3) DURO-TAK 387-2052 was added into the above liquid, followed by mixing and stirring uniformly to provide an intermediate solution.

Step 2: coating and drying to remove solvent. The intermediate solution was coated on a SCOTCHPAK^{™}1022 release film (protective layer), with a wet film thickness of 300 µm, and then dried. Drying was carried out by a three-stage drying method, i.e., drying at 20 °C for 10 min, drying at 60 °C for 10 min, and drying at 75 °C for 10 min, to provide a dried matrix, with a thickness of 120 µm.

Step 3: laminating, compounding and cutting. A SCOTCHPAK^{™}9730 backing film (backing layer) was laminated and compounded with the above dried matrix, and then cut into patches of 10 cm². See FIG. 2 for IVPT results.

### [Example 4] Penetration Enhancer: Glyceryl Monolinoleate

| Ingredient | Formulation Ratio (%) |
|---|---|
| Rasagiline mesylate | 3.00 |
| DURO-TAK 387-2052 | 71.90 |
| Crospovidone | 15.00 |
| Povidone K25 | 2.00 |
| Vitamin E | 0.10 |
| Triethyl citrate | 5.00 |
| Glyceryl monolinoleate | 3.00 |
| Total | 100.00 |
| Solvent (anhydrous ethanol) | 33.00 |

Step 1: preparing a drug-containing adhesive layer (matrix layer). (1) Rasagiline mesylate, vitamin E, triethyl citrate, glyceryl monolinoleate, and anhydrous ethanol were mixed and stirred until all solids were completely dissolved, so as to provide a transparent homogeneous solution; (2) crospovidone was added into the above solution, followed by pre-mixing and stirring uniformly and then homogenizing, to provide a white emulsion liquid; and (3) DURO-TAK 387-2052 was added into the above liquid, followed by mixing and stirring uniformly to provide an intermediate solution.

Step 2: coating and drying to remove solvent. The intermediate solution was coated on a SCOTCHPAK^{™}1022 release film (protective layer), with a wet film thickness of 300 µm, and then dried. Drying was carried out by a three-stage drying method, i.e., drying at 25 °C for 10 min, drying at 50 °C for 8 min, and drying at 80 °C for 5 min, to provide a dried matrix, with a thickness of 120 µm.

Step 3: laminating, compounding and cutting. A SCOTCHPAK^{™}1109 backing film (backing layer) was laminated and compounded with the above dried matrix, and then cut into patches of 10 cm². See FIG. 2 for IVPT results.

### [Example 5] Penetration Enhancer: Glyceryl Monooleate

| Ingredient | Formulation Ratio (%) |
|---|---|
| Rasagiline mesylate | 3.00 |
| DURO-TAK 387-2052 | 71.95 |
| Crospovidone | 15.00 |
| Povidone K25 | 2.00 |
| Vitamin E | 0.05 |
| Triethyl citrate | 5.00 |
| Glyceryl monooleate | 3.00 |
| Total | 100.00 |
| Solvent (anhydrous ethanol) | 28.00 |

Step 1: preparing a drug-containing adhesive layer (matrix layer). (1) Rasagiline mesylate, vitamin E, triethyl citrate, glyceryl monooleate, and anhydrous ethanol were mixed and stirred until all solids were completely dissolved, so as to provide a transparent homogeneous solution; (2) crospovidone was added into the above solution, followed by pre-mixing and stirring uniformly and then homogenizing, to provide a white emulsion liquid; and (3) DURO-TAK 387-4098 was added into the above liquid, followed by mixing and stirring uniformly to provide an intermediate solution.

Step 2: coating and drying to remove solvent. The intermediate solution was coated on a SCOTCHPAK^{™}2054 release film (protective layer), with a wet film thickness of 300 µm, and then dried. Drying was carried out by a three-stage drying method, i.e., drying at 25 °C for 8 min, drying at 55 °C for 10 min, and drying at 80 °C for 5 min, to provide a dried matrix, with a thickness of 120 µm.

Step 3: laminating, compounding and cutting. A SCOTCHPAK^{™}9730 backing film (backing layer) was laminated and compounded with the above dried matrix, and then cut into patches of 10 cm². See FIG. 2 for IVPT results.

### [Example 6] Penetration Enhancer: Polyglyceryl oleate

| Ingredient | Formulation Ratio (%) |
|---|---|
| Rasagiline mesylate | 3.00 |
| DURO-TAK 387-2052 | 71.95 |
| Crospovidone | 15.00 |
| Copovidone VA 64 | 2.00 |
| Vitamin E | 0.05 |
| Triethyl citrate | 5.00 |
| Polyglyceryl oleate | 3.00 |
| Total | 100.00 |
| Anhydrous ethanol | 30.00 |

Step 1: preparing a drug-containing adhesive layer (matrix layer). (1) Rasagiline mesylate, vitamin E, triethyl citrate, polyglyceryl oleate, and anhydrous ethanol were mixed and stirred until all solids were completely dissolved, so as to provide a transparent homogeneous solution; (2) crospovidone was added into the above solution, followed by pre-mixing and stirring uniformly and then homogenizing, to provide a white emulsion liquid; and (3) DURO-TAK 387-2052 was added into the above liquid, followed by mixing and stirring uniformly to provide an intermediate solution.

Step 2: coating and drying to remove solvent. The intermediate solution was coated on a SCOTCHPAK^{™}1022 release film (protective layer), with a wet film thickness of 300 µm, and then dried. Drying was carried out by a three-stage drying method, i.e., drying at 20 °C for 10 min, drying at 65 °C for 8 min, and drying at 75 °C for 10 min, to provide a dried matrix, with a thickness of 120 µm.

Step 3: laminating, compounding and cutting. A SCOTCHPAK^{TM}9738 backing film (backing layer) was laminated and compounded with the above dried matrix, and then cut into patches of 10 cm². See FIG. 2 for IVPT results.

### [Example 7] Penetration Enhancer: Propylene Glycol Monocaprylate

| Ingredient | Formulation Ratio (%) |
|---|---|
| Rasagiline mesylate | 3.00 |
| DURO-TAK 387-2052 | 71.90 |
| Crospovidone | 15.00 |
| Copovidone VA 64 | 2.00 |
| Vitamin E | 0.10 |
| Triethyl citrate | 5.00 |
| Propylene glycol monocaprylate | 3.00 |
| Total | 100.00 |
| Solvent (anhydrous ethanol) | 30.00 |

Step 1: preparing a drug-containing adhesive layer (matrix layer). (1) Rasagiline mesylate, vitamin E, triethyl citrate, propylene glycol monocaprylate, and anhydrous ethanol were mixed and stirred until all solids were completely dissolved, so as to provide a transparent homogeneous solution; (2) crospovidone was added into the above solution, followed by pre-mixing and stirring uniformly and then homogenizing, to provide a white emulsion liquid; and (3) DURO-TAK 387-2287 was added into the above liquid, followed by mixing and stirring uniformly to provide an intermediate solution.

Step 2: coating and drying to remove solvent. The intermediate solution was coated on a SCOTCHPAK^{™}2054 release film (protective layer), with a wet film thickness of 300 µm, and then dried. Drying was carried out by a three-stage drying method, i.e., drying at 25 °C for 10 min, drying at 60 °C for 10 min, and drying at 75 °C for 10 min, to provide a dried matrix, with a thickness of 120 µm.

Step 3: laminating, compounding and cutting. A SCOTCHPAK^{™}1109 backing film (backing layer) was laminated and compounded with the above dried matrix, and then cut into patches of 10 cm². See FIG. 2 for IVPT results.

**Table 1 Q-t Equations of Formulations of [Examples 3-7]**

| Example | Q-t Equation | J (µg/cm²/h) | R² |
|---|---|---|---|
| Example 3 | Y=0.1475X-0.3184 | 0.1475±0.108 | 0.9877 |
| Example 4 | Y=0.9944X-2.9081 | 0.9944±0.121 | 0.9944 |
| Example 5 | Y=0.8527X-2.7615 | 0.8527±0.130 | 0.9870 |
| Example 6 | Y=1.958X-7.613 | 1.9580±0.115 | 0.9596 |
| Example 7 | Y=1.5849X-5.8223 | 1.5849±0.119 | 0.9973 |

In the above, J represents a permeation rate. (Note: R² is a parameter for evaluating a fitting effect of a linear regression model. When the value is 1, it is indicated that all data points of the model completely fall on a regression line, the closer the value is to 1, the better the fitting effect is, and the higher the fitting degree of the model is; and the closer the value is to 0, the worse the fitting effect is, and the lower the model fitting degree is.)

### Analysis of Results of [Examples 3-7]:

Permeation rate: without penetration enhancer (J=0.1475) < glyceryl monooleate Peceol (HLB=1) (J=0.8527) < glyceryl monolinoleate MCC (HLB=1) (J=0.9944) < propylene glycol monocaprylate C90 (HLB=5) (J=1.5849) < polyglyceryl oleate CC497 (HLB=3) (J=1.9380). All formulations added with the penetration enhancer had improved J value to different degrees compared with the formulation without a penetration enhancer. The permeation rates of propylene glycol monocaprylate and polyglyceryl fatty acid ester were increased by a factor of more than 10 and 13, respectively, compared with that without a penetration enhancer. Therefore, the penetration enhancers with HLB values of 3-5 in the present formulation has better penetration-promoting effect, and the penetration enhancers with HLB values of 3-5 are preferred for the rasagiline mesylate patch.

[Examples 8-9] further combined penetration enhancers with different HLB values of 3-5 and plasticizer triethyl citrate to control a delivery rate.

IVRT *in vitro* release in [Examples 8-9] is listed in Table 2. IVPT is shown in FIG. 3. Permeation quantity (Q)-time (t) equations are listed in Table 3.

### [Example 8] Combination of Penetration Enhancers

| Ingredient | Formulation Ratio (%) |
|---|---|
| Rasagiline mesylate | 3.00 |
| DURO-TAK 387-2052 | 69.90 |
| Crospovidone | 15.00 |
| Povidone K25 | 2.00 |
| Vitamin E | 0.10 |
| Triethyl citrate | 4.00 |
| Propylene glycol monocaprylate | 2.00 |
| Polyglyceryl oleate | 4.00 |
| Total | 100.00 |
| Solvent (anhydrous ethanol) | 30.00 |

Step 1: preparing a drug-containing adhesive layer (matrix layer). (1) Rasagiline mesylate, vitamin E, triethyl citrate, propylene glycol monocaprylate, polyglyceryl oleate and anhydrous ethanol were mixed and stirred until all solids were completely dissolved, so as to provide a transparent homogeneous solution; (2) crospovidone was added into the above solution, followed by pre-mixing and stirring uniformly and then homogenizing, to provide a white emulsion liquid; and (3) DURO-TAK 387-2052 was added into the above liquid, followed by mixing and stirring uniformly to provide an intermediate solution.

Step 2: coating and drying to remove solvent. The intermediate solution was coated on a SCOTCHPAK^{™}1022 release film (protective layer), with a wet film thickness of 300 µm, and then dried. Drying was carried out by a three-stage drying method, i.e., drying at 25 °C for 10 min, drying at 65 °C for 8 min, and drying at 80 °C for 5 min, to provide a dried matrix, with a thickness of 120 µm.

Step 3: laminating, compounding and cutting. A SCOTCHPAK^{TM}9738 backing film (backing layer) was laminated and compounded with the above dried matrix, and then cut into patches of 10 cm².

### [Example 9] Combination of Penetration Enhancers

| Ingredient | Formulation Ratio (%) |
|---|---|
| Rasagiline mesylate | 3.00 |
| DURO-TAK 387-2052 | 68.90 |
| Crospovidone | 15.00 |
| Povidone K25 | 2.00 |
| Vitamin E | 0.10 |
| Triethyl citrate | 5.00 |
| Propylene glycol monocaprylate | 2.00 |
| Polyglyceryl oleate | 4.00 |
| Total | 100.00 |
| Anhydrous ethanol | 30.00 |

Step 1: preparing a drug-containing adhesive layer (matrix layer). (1) Rasagiline mesylate, vitamin E, triethyl citrate, propylene glycol monocaprylate, polyglyceryl oleate and anhydrous ethanol were mixed and stirred until all solids were completely dissolved, so as to provide a transparent homogeneous solution; (2) crospovidone was added into the above solution, followed by pre-mixing and stirring uniformly and then homogenizing, to provide a white emulsion liquid; and (3) DURO-TAK 387-2052 was added into the above liquid, followed by mixing and stirring uniformly to provide an intermediate solution.

Step 2: coating and drying to remove solvent. The intermediate solution was coated on a SCOTCHPAK^{™}1022 release film (protective layer), with a wet film thickness of 300 µm, and then dried. Drying was carried out by a three-stage drying method, i.e., drying at 20 °C for 10 min, drying at 60 °C for 10 min, and drying at 75 °C for 10 min, to provide a dried matrix, with a thickness of 120 µm.

Step 3: laminating, compounding and cutting. A SCOTCHPAK^{TM}9738 backing film (backing layer) was laminated and compounded with the above dried matrix, and then cut into patches of 10 cm².

**Table 2 IVRT Results of [Examples 8-9]**

| Time (h) | Release Rate (%) | | Release Amount (ug/cm²) | |
|---|---|---|---|---|
| | Example 8 | Example 9 | Example 8 | Example 9 |
| 0.5 | 33.77 | 40.31 | 119.13 | 118.62 |
| 1 | 49.43 | 54.81 | 167.04 | 161.29 |
| 2 | 64.98 | 68.94 | 217.06 | 202.86 |
| 4 | 77.50 | 80.96 | 257.03 | 238.24 |
| 8 | 86.57 | 89.44 | 286.95 | 263.18 |
| 12 | 90.43 | 92.45 | 300.17 | 272.02 |
| 24 | 93.48 | 94.45 | 311.31 | 277.91 |

**Table 3 Cumulative Permeation Quantity (Q)-Time (t) Relations of [Examples 8-9]**

| Example | Q-t Equation | J (µg/cm²/h) | R² |
|---|---|---|---|
| Example 8 | Y=1.9580X-7.6130 | 1.9580±0.1150 | 0.9596 |
| Example 9 | Y=2.6080X-7.7330 | 2.6080±0.1985 | 0.9766 |

### Analysis of Results of [Examples 8-9]:

IVRT comparison between [Example 8] and [Example 9] reveals that release behaviors of formulation IVRT were substantially consistent, while [Example 9] had a slightly higher release rate than [Example 8]. It is presumed that the penetration enhancer with a low HLB value may be more advantageous for drug release. From comparison between [Example 8] and [Example 9], it can be seen that under a condition of the same total amount of penetration enhancers (6%), the permeation rate with 5% plasticizer is higher than that with 4%. In conjunction with the conclusion that the release rate of [Example 9] is slightly higher than that of [Example 8], it is presumed that an increased proportion of the plasticizer may lead to an increased drug release, and an increased skin drug concentration gradient within the same time is more beneficial to the progress of penetration according to Fick Law; therefore, the formulation with a large release amount also correspondingly has a higher permeation rate.

In addition, in order to further demonstrate that the patch has relatively good use performance, transdermal patches prepared with the formulation and process of [Example 9] are also tested for a parameter as follows:
Peel force: using a testing apparatus under the item of Method 3 "peel strength test" of general chapter 0952 of Volume IV of the *Pharmacopoeia of the People's Republic of China* (2020 edition), and using a 180 °C peel testing method. Operation was repeated and a mean of 6 results was calculated, being 1.15 N/50 mm.

Finally, in order to further demonstrate that the patch has good stability, transdermal patches prepared with the formulation and process of [Example 9] were further tested for the stability, and results are listed in Table 4.

**TABLE 4 Changes in Main Drug Content of Transdermal Patches under Different Evaluation Conditions**

| | | |
|---|---|---|
| Evaluation Condition | 60 °C, 30 days | 40 °C, 30 days |
| Main Drug Content | No change | No change |

The above examples are specific examples corresponding to solution 1 and method 1, and following examples are specific examples corresponding to solution 2 and method 2.

In the following description, [Examples 1'-6'] compared formulations with crospovidone and without crospovidone, aiming at demonstrating that the crospovidone can carry the water-soluble drug rasagiline mesylate, and the final drug content of the system was 0.5-5.0% of rasagiline mesylate (if it is higher, the process is difficult to implement, and cannot be carried out industrially), and an appropriate mass ratio of rasagiline mesylate to crospovidone was 1:5 to 1:15.

[Examples 7'-11'] compared and evaluated formulation IVPT (English abbreviation for *in vitro* permeation testing) without a penetration enhancer and with penetration enhancers having different HLB (English abbreviation for hydrophile lipophilic balance) values, to further demonstrate the necessity of using the penetration enhancer and the reason why polyglycerol fatty acid is preferred. Permeation quantity (Q)-time (t) equations of [Examples 7'-11'] are listed in Table 5.

[Examples 12'-14'] compared and evaluated formulation IVRT (English abbreviation for *in vitro* release testing) containing levulinic acid, caprylic acid, and undecylenic acid, to further demonstrate the reason why levulinic acid is preferred as the release promoter. IVRT results in [Examples 12'-14'] are listed in Table 6.

[Examples 15'-17'] evaluated IVPT through combinations of penetration enhancer and release promoter in different proportions, to further demonstrate that controlled drug delivery rates can be achieved. IVRT and IVPT results are listed in Table 7 and Table 8.

In the present disclosure, research of *in vitro* release testing (IVRT) adopts method 4 (paddle over disk) of 0931 of the *Pharmacopoeia of the People's Republic of China* (2020 edition). Dissolution instrument and high performance liquid chromatography are utilized to calculate a cumulative release amount within a certain sampling time, and release rate (%)-time (t) relation curves of rasagiline are plotted. Implementation solution of IVRT is as follows:
Release medium: pH 4.5 acetate buffer;
Medium volume/temperature/rotational speed/sample amount: 900 mL, 32±0.5 °C, 50 r/min, 3 parts per batch;
Sampling time points: 0.5 h, 1 h, 2 h, 4 h, 8 h, 12 h, 18 h, and 24 h;
Chromatography conditions are: chromatographic column: Agilent Zorbax SB-C18 4.6×250 mm, 5 µm; mobile phase: 10 mM KH2PO4: ACN=80:20; flow rate: 1.0 ml/min; column temperature: 40 °C; detection wavelength: 210 nm; injection volume: 50 µl; and collection time: 6 min.

Research of *in vitro* permeation testing (IVPT) utilized Franz cell (*in vitro* transdermal experimental system) and Bama miniature pig skin to assess permeability of rasagiline mesylate. A receiving solution was 0.01% gentamicin sulfate (pH 7.4 PBS). Contents of rasagiline at different sampling points in a receiving tank were determined using high performance liquid chromatography, a cumulative permeation quantity of rasagiline was calculated, and cumulative permeation quantity (Q)-time (t) relation curves were plotted.

In addition, in order to further demonstrate that the patch has relatively good use performance, the transdermal patches prepared with the formulation and process of [Example 15'] were further tested for adhesive force and peel force, and results are listed in Table 9.

Finally, in order to further demonstrate that the patch has good stability, the transdermal patches prepared with the formulation and process of [Example 15'] were further tested for stability. Results are listed in Table 10.

### [Examples 1'-6']

The key innovative concept of the present disclosure lies in carrying the water-soluble drug rasagiline mesylate by incorporating PVPP so as to yield patches with high drug load. For this purpose, an experiment was designed to evaluate three variables, i.e., whether to add PVPP, an amount of the PVPP added, and a ratio of PVPP to rasagiline mesylate, to explore a final formulation scheme.

Experimental design is listed in the table below.

| Ingredient | Example 1' | Example 2' | Example 3' | Example 4' | Example 5' | Example 6' |
|---|---|---|---|---|---|---|
| | Mass Ratio % | | | | | |
| Rasagiline mesylate | 0.10 | 0.50 | 2.00 | 0.50 | 2.00 | 5.00 |
| Crospovidone | 0.00 | 0.00 | 0.00 | 7.50 | 15.00 | 25.00 |
| DURO-TAK 387-2052 | 93.90 | 93.50 | 92.00 | 86.00 | 77.00 | 64.00 |
| Triethyl citrate | 1.00 | 1.00 | 1.00 | 1.00 | 1.00 | 1.00 |
| Polyglyceryl oleate | 5.00 | 5.00 | 5.00 | 5.00 | 5.00 | 5.00 |
| Total (100 g in total, the same below) | 100.00 | 100.00 | 100.00 | 100.00 | 100.00 | 100.00 |
| Anhydrous ethanol (in g, the same below) | 33.00 | 33.00 | 33.00 | 33.00 | 33.00 | 33.00 |
| Crystallization status after placement at room temperature for 72 hours | Crystallization, with a crystallization area of about 8.07% | Crystallization, with a crystallization area of about 11.52% | Crystallization, with a crystallization area of about 36.71% | No crystallization | No crystallization | No crystallization |

Step 1: preparing a drug-containing adhesive layer (matrix layer). (1) Rasagiline mesylate, triethyl citrate, polyglyceryl oleate, and anhydrous ethanol were mixed and stirred until all solids were completely dissolved, so as to provide a transparent homogeneous solution; and (2) DURO-TAK 387-2052 was added into the above liquid, followed by mixing and stirring uniformly to provide an intermediate solution.

Step 2: coating and drying to remove solvent. The intermediate solution was coated on a SCOTCHPAK^{™}2504 protective layer, with a wet film thickness of 300 µm, and then dried. Drying was carried out by a three-stage drying method, i.e., drying at 30 °C for 8 min, drying at 65 °C for 8 min, and drying at 80 °C for 10 min, to provide a dried matrix, with a thickness of 120 µm.

Step 3: laminating, compounding and cutting. A SCOTCHPAK^{™}1109 backing layer was laminated and compounded with the above dried matrix, and then cut into patches of 10 cm².

After the patches prepared by the above formulation and process were left at room temperature for 3 days, it was observed under a polarizing microscope whether there was a crystallization phenomenon.

Comparison between Examples 1' and 3' can prove that the crystallization phenomena occurred in the absence of PVPP no matter in the case of high drug content (2%) or low drug content (0.1%), and quantitative analysis and statistics were performed on the crystallization amount (see FIG. 4). The extent of crystallization is calculated as percentage of crystallization area relative to a total area. No crystallization was observed in Examples 4'-6', and microscopic morphology observed without polarized light and microscopic morphology under polarized light were presented in parallel for comparison.

Examples 4'-6' revealed that incorporation of PVPP can avoid crystallization. At the same time, a suitable ratio of an amount of PVPP added to drug was explored, and a maximum drug content of the system is no more than 5.0.

Subsequent examples will further address the problems of penetration, release, and stable storage.

[Examples 7'-11'] further compared and evaluated formulation IVP without a penetration enhancer and with penetration enhancers having different HLB values, to further demonstrate the necessity of using the penetration enhancer and the reason why polyglycerol fatty acid is preferred. Permeation quantity (Q)-time (t) equations of [Examples 7'-11'] are listed in Table 5.

### [Example' 7] Without Penetration Enhancer

| Ingredient | Formulation Ratio (%) |
|---|---|
| Rasagiline mesylate | 1.75 |
| DURO-TAK 387-2052 | 75.15 |
| Crospovidone | 17.50 |
| Vitamin E | 0.10 |
| Triethyl citrate | 5.00 |
| Levulinic acid | 0.50 |
| Total | 100.00 |
| Solvent (anhydrous ethanol) | 33.00 |

Step 1: preparing a drug-containing adhesive layer (matrix layer). (1) Rasagiline mesylate, vitamin E, triethyl citrate, levulinic acid, and anhydrous ethanol were mixed and stirred until all solids were completely dissolved, so as to provide a transparent homogeneous solution; (2) crospovidone was added into the above solution, followed by pre-mixing and stirring uniformly and then homogenizing, to provide a white emulsion liquid; and (3) DURO-TAK 387-2052 was added into the above liquid, followed by mixing and stirring uniformly to provide an intermediate solution.

Step 2: coating and drying to remove solvent. The intermediate solution was coated on a SCOTCHPAK^{™}1022 protective layer, with a wet film thickness of 300 µm, and then dried. Drying was carried out by a three-stage drying method, i.e., drying at 25 °C for 10 min, drying at 60 °C for 10 min, and drying at 75 °C for 10 min, to provide a dried matrix, with a thickness of 120 µm.

Step 3: laminating, compounding and cutting. A SCOTCHPAK^{TM}9738 backing layer was laminated and compounded with the above dried matrix, and then cut into patches of 10 cm².

### [Example 8'] Penetration Enhancer: Glyceryl Monolinoleate

| Ingredient | Formulation Ratio (%) |
|---|---|
| Rasagiline mesylate | 1.75 |
| DURO-TAK 387-2052 | 72.15 |
| Crospovidone | 17.50 |
| Vitamin E | 0.10 |
| Triethyl citrate | 5.00 |
| Glyceryl monolinoleate | 3.00 |
| Levulinic acid | 0.50 |
| Total | 100.00 |
| Solvent (anhydrous ethanol) | 33.00 |

Step 1: preparing a drug-containing adhesive layer (matrix layer). (1) Rasagiline mesylate, vitamin E, triethyl citrate, glyceryl monolinoleate, levulinic acid, and anhydrous ethanol were mixed and stirred until all solids were completely dissolved, so as to provide a transparent homogeneous solution; (2) crospovidone was added into the above solution, followed by pre-mixing and stirring uniformly and then homogenizing, to provide a white emulsion liquid; and (3) DURO-TAK 387-2052 was added into the above liquid, followed by mixing and stirring uniformly to provide an intermediate solution.

Step 2: coating and drying to remove solvent. The intermediate solution was coated on a SCOTCHPAK^{™}1022 protective layer, with a wet film thickness of 300 µm, and then dried. Drying was carried out by a three-stage drying method, i.e., drying at 25 °C for 10 min, drying at 60 °C for 10 min, and drying at 75 °C for 10 min, to provide a dried matrix, with a thickness of 120 µm.

Step 3: laminating, compounding and cutting. A SCOTCHPAK^{™}9738 backing film was laminated and compounded with the above dried matrix, and then cut into patches of 10 cm².

### [Example 9'] Penetration Enhancer: Glyceryl Monooleate

| Ingredient | Formulation Ratio (%) |
|---|---|
| Rasagiline mesylate | 1.75 |
| DURO-TAK 387-2052 | 72.15 |
| Crospovidone | 17.50 |
| Vitamin E | 0.10 |
| Triethyl citrate | 5.00 |
| Glyceryl monooleate | 3.00 |
| Levulinic acid | 0.50 |
| Total | 100.00 |
| Solvent (anhydrous ethanol) | 33.00 |

Step 1: preparing a drug-containing adhesive layer (matrix layer). (1) Rasagiline mesylate, vitamin E, triethyl citrate, glyceryl monooleate, levulinic acid, and anhydrous ethanol were mixed and stirred until all solids were completely dissolved, so as to provide a transparent homogeneous solution; (2) crospovidone was added into the above solution, followed by pre-mixing and stirring uniformly and then homogenizing, to provide a white emulsion liquid; and (3) DURO-TAK 387-2052 was added into the above liquid, followed by mixing and stirring uniformly to provide an intermediate solution.

Step 2: coating and drying to remove solvent. The intermediate solution was coated on a SCOTCHPAK^{™}1022 protective layer, with a wet film thickness of 300 µm, and then dried. Drying was carried out by a three-stage drying method, i.e., drying at 25 °C for 10 min, drying at 60 °C for 10 min, and drying at 75 °C for 10 min, to provide a dried matrix, with a thickness of 120 µm.

Step 3: laminating, compounding and cutting. A SCOTCHPAK^{TM}9738 backing layer was laminated and compounded with the above dried matrix, and then cut into patches of 10 cm².

### [Example 10'] Penetration Enhancer: Polyglyceryl oleate

| Ingredient | Formulation Ratio (%) |
|---|---|
| Rasagiline mesylate | 1.75 |
| DURO-TAK 387-2052 | 72.15 |
| Crospovidone | 17.50 |
| Vitamin E | 0.10 |
| Triethyl citrate | 5.00 |
| Polyglyceryl oleate | 3.00 |
| Levulinic acid | 0.50 |
| Total | 100.00 |
| Anhydrous ethanol | 33.00 |

Step 1: preparing a drug-containing adhesive layer (matrix layer). (1) Rasagiline mesylate, vitamin E, triethyl citrate, polyglyceryl oleate, levulinic acid, and anhydrous ethanol were mixed and stirred until all solids were completely dissolved, so as to provide a transparent homogeneous solution; (2) crospovidone was added into the above solution, followed by pre-mixing and stirring uniformly and then homogenizing, to provide a white emulsion liquid; and (3) DURO-TAK 387-2052 was added into the above liquid, followed by mixing and stirring uniformly to provide an intermediate solution.

Step 2: coating and drying to remove solvent. The intermediate solution was coated on a SCOTCHPAK^{™}1022 protective layer, with a wet film thickness of 300 µm, and then dried. Drying was carried out by a three-stage drying method, i.e., drying at 25 °C for 10 min, drying at 60 °C for 10 min, and drying at 75 °C for 10 min, to provide a dried matrix, with a thickness of 120 µm.

Step 3: laminating, compounding and cutting. A SCOTCHPAK^{TM}9738 backing layer was laminated and compounded with the above dried matrix, and then cut into patches of 10 cm².

### [Example 11'] Penetration Enhancer: Propylene Glycol Monocaprylate

| Ingredient | Formulation Ratio (%) |
|---|---|
| Rasagiline mesylate | 1.75 |
| DURO-TAK 387-2052 | 72.15 |
| Crospovidone | 17.50 |
| Vitamin E | 0.10 |
| Triethyl citrate | 5.00 |
| Propylene glycol monocaprylate | 3.00 |
| Levulinic acid | 0.50 |
| Total | 100.00 |
| Solvent (anhydrous ethanol) | 33.00 |

Step 1: preparing a drug-containing adhesive layer (matrix layer). (1) Rasagiline mesylate, vitamin E, triethyl citrate, propylene glycol monocaprylate, levulinic acid, and anhydrous ethanol were mixed and stirred until all solids were completely dissolved, so as to provide a transparent homogeneous solution; (2) crospovidone was added into the above solution, followed by pre-mixing and stirring uniformly and then homogenizing, to provide a white emulsion liquid; and (3) DURO-TAK 387-2052 was added into the above liquid, followed by mixing and stirring uniformly to provide an intermediate solution.

Step 2: coating and drying to remove solvent. The intermediate solution was coated on a SCOTCHPAK^{™}1022 protective layer, with a wet film thickness of 300 µm, and then dried. Drying was carried out by a three-stage drying method, i.e., drying at 25 °C for 10 min, drying at 60 °C for 10 min, and drying at 75 °C for 10 min, to provide a dried matrix, with a thickness of 120 µm.

Step 3: laminating, compounding and cutting. A SCOTCHPAK^{TM}9738 backing layer was laminated and compounded with the above dried matrix, and then cut into patches of 10 cm².

**Table 5 Q-t Equations of Formulations of [Examples 7'-11']**

| Example | Q-t Equation | J (µg/cm²/h) | R² |
|---|---|---|---|
| Example 7' | Y=0.1485X-0.3091 | 0.1485±0.118 | 0.9897 |
| Example 8' | Y=0.9894X-2.8091 | 0.9894±0.103 | 0.9984 |
| Example 9' | Y=0.8573X-2.1675 | 0.8573±0.107 | 0.9890 |
| Example 10' | Y=1.9807X-6.1347 | 1.9807±0.113 | 0.9798 |
| Example 11' | Y=1.5948X-5.2283 | 1.5948±0.109 | 0.9976 |

In the above, J represents a permeation rate. (Note: R² is a parameter for evaluating a fitting effect of a linear regression model. When the value is 1, it is indicated that all data points of the model completely fall on a regression line, the closer the value is to 1, the better the fitting effect is, and the higher the fitting degree of the model is; and the closer the value is to 0, the worse the fitting effect is, and the lower the model fitting degree is.)

### Analysis of Results of [Examples 7'-11']:

All formulations added with the penetration enhancer had improved J value to different degrees compared with the formulation without a penetration enhancer. The penetration-promoting effect of the polyglyceryl fatty acid ester is the best, and the rasagiline mesylate patch preferably uses polyglyceryl fatty acid ester as the penetration enhancer.

[Examples 12'-14'] compared and evaluated formulation IVRT with levulinic acid, caprylic acid, and undecylenic acid, to further demonstrate the reason why levulinic acid is preferred as the release promoter. IVRT results are listed in Table 6.

### [Example 12'] Release Promoter: Levulinic Acid

| Ingredient | Formulation Ratio (%) |
|---|---|
| Rasagiline mesylate | 1.75 |
| DURO-TAK 387-2052 | 69.15 |
| Crospovidone | 17.50 |
| Vitamin E | 0.10 |
| Triethyl citrate | 7.00 |
| Polyglycerol fatty acid | 4.00 |
| Levulinic acid | 0.50 |
| Total | 100.00 |
| Solvent (anhydrous ethanol) | 33.00 |

Step 1: preparing a drug-containing adhesive layer (matrix layer). (1) Rasagiline mesylate, vitamin E, triethyl citrate, polyglyceryl fatty acid ester, levulinic acid, and anhydrous ethanol were mixed and stirred until all solids were completely dissolved, so as to provide a transparent homogeneous solution; (2) crospovidone was added into the above solution, followed by pre-mixing and stirring uniformly and then homogenizing, to provide a white emulsion liquid; and (3) DURO-TAK 387-2052 was added into the above liquid, followed by mixing and stirring uniformly to provide an intermediate solution.

Step 2: coating and drying to remove solvent. The intermediate solution was coated on a 75E-0010BD protective layer, with a wet film thickness of 300 µm, and then dried. Drying was carried out by a three-stage drying method, i.e., drying at 25 °C for 10 min, drying at 65 °C for 8 min, and drying at 80 °C for 8 min, to provide a dried matrix, with a thickness of 120 µm.

Step 3: laminating, compounding and cutting. A SCOTCHPAK^{TM}9738 backing layer was laminated and compounded with the above dried matrix, and then cut into patches of 10 cm². IVRT results are listed in Table 6.

### [Example 13'] Release Promoter: Caprylic Acid

| Ingredient | Formulation Ratio (%) |
|---|---|
| Rasagiline mesylate | 1.75 |
| DURO-TAK 387-2052 | 69.15 |
| Crospovidone | 17.50 |
| Vitamin E | 0.10 |
| Triethyl citrate | 7.00 |
| Polyglycerol fatty acid | 4.00 |
| Caprylic acid | 0.50 |
| Total | 100.00 |
| Solvent (anhydrous ethanol) | 33.00 |

Step 1: preparing a drug-containing adhesive layer (matrix layer). (1) Rasagiline mesylate, vitamin E, triethyl citrate, polyglyceryl fatty acid ester, caprylic acid, and anhydrous ethanol were mixed and stirred until all solids were completely dissolved, so as to provide a transparent homogeneous solution; (2) crospovidone was added into the above solution, followed by pre-mixing and stirring uniformly and then homogenizing, to provide a white emulsion liquid; and (3) DURO-TAK 387-2052 was added into the above liquid, followed by mixing and stirring uniformly to provide an intermediate solution.

Step 2: coating and drying to remove solvent. The intermediate solution was coated on a 75E-0010BD protective layer, with a wet film thickness of 300 µm, and then dried. Drying was carried out by a three-stage drying method, i.e., drying at 25 °C for 10 min, drying at 65 °C for 8 min, and drying at 80 °C for 8 min, to provide a dried matrix, with a thickness of 120 µm.

Step 3: laminating, compounding and cutting. A SCOTCHPAK^{™}9738 backing layer was laminated and compounded with the above dried matrix, and then cut into patches of 10 cm². IVRT results are listed in Table 6.

### [Example 14'] Release Promoter: Undecylenic Acid

| Ingredient | Formulation Ratio (%) |
|---|---|
| Rasagiline mesylate | 1.75 |
| DURO-TAK 387-2052 | 69.15 |
| Crospovidone | 17.50 |
| Vitamin E | 0.10 |
| Triethyl citrate | 7.00 |
| Polyglycerol fatty acid ester | 4.00 |
| Undecylenic acid | 0.50 |
| Total | 100.00 |
| Solvent (anhydrous ethanol) | 33.00 |

Step 1: preparing a drug-containing adhesive layer (matrix layer). (1) Rasagiline mesylate, vitamin E, triethyl citrate, polyglyceryl fatty acid ester, undecylenic acid, and anhydrous ethanol were mixed and stirred until all solids were completely dissolved, so as to provide a transparent homogeneous solution; (2) crospovidone was added into the above solution, followed by pre-mixing and stirring uniformly and then homogenizing, to provide a white emulsion liquid; and (3) DURO-TAK 387-2052 was added into the above liquid, followed by mixing and stirring uniformly to provide an intermediate solution.

Step 2: coating and drying to remove solvent. The intermediate solution was coated on a 75E-0010BD protective layer, with a wet film thickness of 300 µm, and then dried. Drying was carried out by a three-stage drying method, i.e., drying at 25 °C for 10 min, drying at 65 °C for 8 min, and drying at 80 °C for 8 min, to provide a dried matrix, with a thickness of 120 µm.

Step 3: laminating, compounding and cutting. A SCOTCHPAK^{TM}9738 backing layer was laminated and compounded with the above dried matrix, and then cut into patches of 10 cm². IVRT results are listed in Table 6.

**Table 6 [Examples 12'-14'] IVRT**

| Example | Release Rate (%) | | | | | | | | |
|---|---|---|---|---|---|---|---|---|---|
| | 0.25 h | 0.5 h | 1 h | 2 h | 4h | 8 h | 12 h | 18 h | 24 h |
| Example 12' | 47.48 | 68.30 | 81.66 | 90.94 | 95.80 | 96.78 | 96.70 | 96.71 | 96.77 |
| Example 13' | 41.28 | 64.17 | 80.41 | 90.82 | 96.45 | 97.51 | 97.33 | 97.71 | 97.76 |
| Example 14' | 39.31 | 64.11 | 79.76 | 90.26 | 96.03 | 97.41 | 97.38 | 97.45 | 97.50 |

Analysis of results of [Examples 12'-14']: during the initial 2 h period, the release rate showed Example 12' (levulinic acid) > Example 13' (caprylic acid) > Example 14' (undecylenic acid), and the three gradually tended to be equivalent in later stages. Therefore, levulinic acid has a strong release-promoting effect in an early stage.

[Examples 15'-17'] evaluated IVPT through combinations of penetration enhancer and release promoter in different proportions, to further demonstrate that controlled drug delivery rates can be achieved. IVRT and IVPT results are listed in Table 7 and Table 8.

### [Example 15']

| Ingredient | Formulation Ratio (%) |
|---|---|
| Rasagiline mesylate | 1.75 |
| DURO-TAK 387-2052 | 73.15 |
| Crospovidone | 17.50 |
| Vitamin E | 0.10 |
| Triethyl citrate | 2.00 |
| Polyglycerol fatty acid ester | 5.00 |
| Levulinic acid | 0.50 |
| Total | 100.00 |
| Solvent (anhydrous ethanol) | 33.00 |

Step 1: preparing a drug-containing adhesive layer (matrix layer). (1) Rasagiline mesylate, vitamin E, triethyl citrate, polyglyceryl fatty acid ester, levulinic acid, and anhydrous ethanol were mixed and stirred until all solids were completely dissolved, so as to provide a transparent homogeneous solution; (2) crospovidone was added into the above solution, followed by pre-mixing and stirring uniformly and then homogenizing, to provide a white emulsion liquid; and (3) DURO-TAK 387-2052 was added into the above liquid, followed by mixing and stirring uniformly to provide an intermediate solution.

Step 2: coating and drying to remove solvent. The intermediate solution was coated on a 75E-0010BD protective layer, with a wet film thickness of 300 µm, and then dried. Drying was carried out by a three-stage drying method, i.e., drying at 25 °C for 10 min, drying at 60 °C for 10 min, and drying at 80 °C for 8 min, to provide a dried matrix, with a thickness of 120 µm.

Step 3: laminating, compounding and cutting. A SCOTCHPAK^{™}1109 backing layer was laminated and compounded with the above dried matrix, and then cut into patches of 10 cm². IVRT and IVPT results are listed in Table 7 and Table 8.

### [Example 16']

| Ingredient | Formulation Ratio (%) |
|---|---|
| Rasagiline mesylate | 1.75 |
| DURO-TAK 387-2052 | 73.65 |
| Crospovidone | 17.50 |
| Vitamin E | 0.10 |
| Triethyl citrate | 2.00 |
| Polyglycerol fatty acid | 4.00 |
| Levulinic acid | 1.00 |
| Total | 100.00 |
| Solvent (anhydrous ethanol) | 33.00 |

Step 1: preparing a drug-containing adhesive layer (matrix layer). (1) Rasagiline mesylate, vitamin E, triethyl citrate, polyglyceryl fatty acid ester, levulinic acid, and anhydrous ethanol were mixed and stirred until all solids were completely dissolved, so as to provide a transparent homogeneous solution; (2) crospovidone was added into the above solution, followed by pre-mixing and stirring uniformly and then homogenizing, to provide a white emulsion liquid; and (3) DURO-TAK 387-2052 was added into the above liquid, followed by mixing and stirring uniformly to provide an intermediate solution.

Step 2: coating and drying to remove solvent. The intermediate solution was coated on a 75E-0010BD protective layer, with a wet film thickness of 300 µm, and then dried. Drying was carried out by a three-stage drying method, i.e., drying at 25 °C for 10 min, drying at 60 °C for 10 min, and drying at 80 °C for 8 min, to provide a dried matrix, with a thickness of 120 µm.

Step 3: laminating, compounding and cutting. A SCOTCHPAK^{™}1109 backing layer was laminated and compounded with the above dried matrix, and then cut into patches of 10 cm². IVRT and IVPT results are listed in Table 7 and Table 8.

### [Example 17']

| Ingredient | Formulation Ratio (%) |
|---|---|
| Rasagiline mesylate | 1.75 |
| DURO-TAK 387-2052 | 74.15 |
| Crospovidone | 17.50 |
| Vitamin E | 0.10 |
| Triethyl citrate | 2.00 |
| Polyglycerol fatty acid | 3.00 |
| Levulinic acid | 1.50 |
| Total | 100.00 |
| Solvent (anhydrous ethanol) | 33.00 |

Step 1: preparing a drug-containing adhesive layer (matrix layer). (1) Rasagiline mesylate, vitamin E, triethyl citrate, polyglyceryl fatty acid ester, levulinic acid, and anhydrous ethanol were mixed and stirred until all solids were completely dissolved, so as to provide a transparent homogeneous solution; (2) crospovidone was added into the above solution, followed by pre-mixing and stirring uniformly and then homogenizing, to provide a white emulsion liquid; and (3) DURO-TAK 387-2052 was added into the above liquid, followed by mixing and stirring uniformly to provide an intermediate solution.

Step 2: coating and drying to remove solvent. The intermediate solution was coated on a 75E-0010BD protective layer, with a wet film thickness of 300 µm, and then dried. Drying was carried out by a three-stage drying method, i.e., drying at 25 °C for 10 min, drying at 60 °C for 10 min, and drying at 80 °C for 8 min, to provide a dried matrix, with a thickness of 120 µm.

Step 3: laminating, compounding and cutting. A SCOTCHPAK^{™}1109 backing layer was laminated and compounded with the above dried matrix, and then cut into patches of 10 cm². IVRT and IVPT results are listed in Table 7 and Table 8.

**Table 7 [Examples 15'-17'] IVRT**

| Example | Release Rate (%) | | | | | | | | |
|---|---|---|---|---|---|---|---|---|---|
| | 0.25 h | 0.5 h | 1 h | 2 h | 4h | 8 h | 12 h | 18h | 24 h |
| Example 15' | 29.40 | 46.38 | 65.65 | 82.46 | 93.35 | 97.43 | 97.76 | 97.59 | 97.83 |
| Example 16' | 30.78 | 47.30 | 65.80 | 82.80 | 94.10 | 98.59 | 99.04 | 98.86 | 98.95 |
| Example 17' | 32.13 | 48.85 | 66.67 | 82.82 | 93.77 | 98.36 | 98.90 | 98.70 | 98.77 |

**Table 8 Cumulative Permeation Quantity (Q)-Time (t) Relations of [Examples 15'-17']**

| Example | Q-t Equation | J (µg/cm²/h) | R² |
|---|---|---|---|
| Example 15' | 1.9166X-9.7164 | 1.9166 | 0.9994 |
| Example 16' | 1.8821X-6.4265 | 1.8821 | 0.9997 |
| Example 17' | 1.7789X+1.0644 | 1.7789 | 0.9972 |

Analysis of results of [Examples 15'-17']: in Examples 15'-17', a release rate was increased with increase of levulinic acid, and the permeation rate was decreased gradually with decrease of polyglyceryl fatty acid ester, further demonstrating the release-promoting effect of levulinic acid and the penetration-promoting effect of polyglyceryl fatty acid ester. The release rate and permeation rate of the drug can be regulated by adjusting amounts of the release promoter and the penetration enhancer.

In addition, in order to further demonstrate that the patches have relatively good use performance, transdermal patches prepared with the formulation and process of [Example 15'] were further tested for peel strength and shear adhesion.

Finally, in order to further demonstrate that the patches have good stability, the transdermal patches prepared with the formulation and process of [Example 15'] were further tested for stability. Results are listed in Table 9.

**Table 9 Test Results of Peel Strength and Shear Adhesion of [Example 15']**

| 180 ° Peel Strength | Shear Adhesion |
|---|---|
| 0.266 N/50mm | 157 min |

Finally, in order to further demonstrate that the patches have good stability, the transdermal patches prepared with the formulation and process of [Example 15'] were further tested for stability. Results are listed in Table 10.

**TABLE 10 Changes in Main Drug Content of Transdermal Patches under Different Evaluation Conditions**

| Evaluation Condition | High Temperature 60 °C | High Temperature 40 °C | High Humidity 92.5% | Lighting 5000 I×1.7 W·m²/h |
|---|---|---|---|---|
| Main Drug Content | 100.68% | 100.93% | 100.77% | 100.53% |

Those skilled in the art should understand that, in the process of preparing the transdermal patch in the present disclosure, the product obtained by the process of step 1 of preparing the drug-containing adhesive layer (the matrix layer and matrix mixture) is an intermediate product: the drug-containing adhesive layer (the matrix layer and matrix mixture), the process thereof can be added with an organic solvent (organic solvent, such as ethanol or isopropanol, which could be easily removed by evaporation) to promote dissolving, dilute and reduce viscosity of the mixture so as to facilitate stirring and subsequent transfer preparation process; alternatively, the organic solvent may not be added (a binder, such as an acrylate pressure-sensitive adhesive, which is liquid at room temperature and under normal pressure); therefore, the intermediate product prepared in the above, the drug-containing adhesive layer (the matrix layer, the matrix mixture), may or may not contain the abovementioned organic solvent. During the preparation process of the present disclosure, due to the addition of the organic solvent can promote dissolving, dilute and reduce the viscosity of the mixture so as to facilitate stirring and subsequent transfer preparation process, the organic solvent of ethanol is added.

Those skilled in the art should understand that after the solvent removal process in step 2, the finally prepared transdermal patch should contain no or minimal residual organic solvent complying with requirements of pharmacopeia or pharmaceutical quality.

Those skilled in the art should understand that the embodiments of the present disclosure described in the above are only illustrative and do not limit the present disclosure. The objective of the present disclosure has been achieved entirely and effectively. The functional and structural principles of the present disclosure have been illustrated and described in the embodiments, and without departing from the principle, the embodiments of the present disclosure can be subjected to any variation or modification.

## Claims

1. A transdermal patch comprising rasagiline mesylate, comprising a backing layer, a matrix layer and a protective layer, wherein the matrix layer comprises following components in mass percentage: 0.5-5.0% of rasagiline mesylate, 40-80% of a binder, 3-25% of crospovidone, 1-5% of a crystallization inhibitor, 1-15% of a penetration enhancer, 1-10% of a plasticizer, and 0.001-0.5% of an antioxidant, wherein a mass ratio of rasagiline mesylate to crospovidone is 1:5 to 1:15, and a content of the binder is finally adjusted to formulate a mass percentage of 100%, wherein the matrix layer further comprises following component in mass percentage: 0.1-5.0% of a release promoter, wherein the release promoter is selected from one or a combination of several of levulinic acid, octanoic acid, and undecanoic acid.

2. A transdermal patch comprising rasagiline mesylate, comprising a backing layer, a matrix layer and a protective layer, wherein the matrix layer comprises following components in mass percentage: 0.5-5.0% of rasagiline mesylate, 40-80% of a binder, 3-25% of crospovidone, 0.1-5.0% of a release promoter, 1-15% of a penetration enhancer, 0.5-10% of a plasticizer, and 0.001-0.5% of an antioxidant, wherein a mass ratio of rasagiline mesylate to crospovidone is 1:5 to 1:15, and a content of the binder is finally adjusted to formulate a mass percentage of 100%, and the release promoter is selected from one or a combination of several of levulinic acid, octanoic acid, and undecanoic acid.

3. The transdermal patch comprising rasagiline mesylate according to claim 1 or 2, wherein the release promoter is levulinic acid.

4. The transdermal patch comprising rasagiline mesylate according to claim 1, wherein a mass ratio of rasagiline mesylate to the crystallization inhibitor is 3:1-3:5.

5. The transdermal patch comprising rasagiline mesylate according to claim 1, wherein a mass ratio of the plasticizer to the penetration enhancer is 1:1-1:5.

6. The transdermal patch comprising rasagiline mesylate according to claim 2, wherein a mass ratio of the release promoter to rasagiline mesylate is 1:1-1:4.

7. The transdermal patch comprising rasagiline mesylate according to claim 2, wherein a mass ratio of the plasticizer to the penetration enhancer is 1:1-1:10.

8. The transdermal patch comprising rasagiline mesylate according to claim 2, wherein a mass ratio of the release promoter to the penetration enhancer is 1:1-1:15.

9. The transdermal patch comprising rasagiline mesylate according to claim 1 or 2, wherein the binder is selected from acrylate pressure-sensitive adhesive.

10. The transdermal patch comprising rasagiline mesylate according to claim 9, wherein the acrylate pressure-sensitive adhesive is selected from one or a combination of several of DURO-TAK 387-2516, DURO-TAK 387-2052, DOR0-TAK 87-4098 and DURO-TAK 387-2287.

11. The transdermal patch comprising rasagiline mesylate according to claim 1, wherein the crystallization inhibitor is at least one substance selected from the group consisting of povidone K90, povidone K30, povidone K25, povidone K17, povidone K12, and copovidone VA 64.

12. The transdermal patch comprising rasagiline mesylate according to claim 1, wherein the crystallization inhibitor is selected from one or a combination of several of povidone K25, povidone K17, povidone K12 and copovidone VA 64.

13. The transdermal patch comprising rasagiline mesylate according to claim 1 or 2, wherein the penetration enhancer is selected from at least one of following substances: propylene glycol monocaprylate, polyglyceryl fatty acid ester, glyceryl monooleate, glyceryl monolinoleate, glyceryl monostearate, Span 80 and Span 60.

14. The transdermal patch comprising rasagiline mesylate according to claim 13, wherein the penetration enhancer is selected from propylene glycol monocaprylate or polyglyceryl fatty acid ester.

15. The transdermal patch comprising rasagiline mesylate according to claim 1 or 2, wherein the plasticizer is selected from one or a combination of several of triethyl citrate, glyceryl triacetate and dibutyl phthalate.

16. The transdermal patch comprising rasagiline mesylate according to claim 15, wherein the plasticizer is selected from triethyl citrate.

17. The transdermal patch comprising rasagiline mesylate according to claim 1 or 2, wherein the antioxidant is selected from one or more of vitamin E (tocopherol), tocopheryl acetate, tocopheryl succinate, tocopheryl nicotinate, tocopheryl palmitate, tocopheryl linoleate and tocopheryl phosphate.

18. The transdermal patch comprising rasagiline mesylate according to claim 17, wherein the antioxidant is selected from vitamin E (tocopherol).

19. The transdermal patch comprising rasagiline mesylate according to claim 1 or 2, wherein the backing layer is selected from one of SCOTCHPAK^{™}9738, SCOTCHPAK^{™}9730, and SCOTCHPAK^{™}1109; and the protective layer is selected from one of SCOTCHPAK^{™}1022, SCOTCHPAK^{™}2504, and 75E-0010BD.

20. A preparation method for a transdermal patch comprising rasagiline mesylate, comprising:
step 1: preparing a drug-containing matrix layer, wherein 0.5-5.0% of rasagiline mesylate, 1-5% of a crystallization inhibitor, 1-15% of a penetration enhancer, 0.1-5.0% of a release promoter, 1-10% of a plasticizer, and 0.001-0.5% of an antioxidant are weighed, dissolved in a solvent, and mixed and stirred until all solids are completely dissolved to provide a homogeneous solution; 3-25% of crospovidone is added into the homogeneous solution, followed by pre-mixing and swelling sufficiently and then homogenizing, to provide a white emulsion liquid; and 40-80% of a binder is added to the white emulsion liquid, followed by mixing and stirring uniformly to provide an intermediate solution;
step 2: coating and drying to remove the solvent, wherein the intermediate solution is coated on a protective layer, with a wet film thickness of 100-500 µm, and then dried to provide a dried matrix, with a dried film thickness of 20-250 µm; and
step 3: laminating, compounding and cutting, wherein the backing layer is laminated and compounded with the dried matrix, and then cut into patches,
wherein the release promoter is selected from one or a combination of several of levulinic acid, octanoic acid, and undecanoic acid, a mass ratio of rasagiline mesylate to crospovidone is 1:5 to 1:15, and a content of the binder is finally adjusted to formulate a mass percentage of 100%.

21. A preparation method for a transdermal patch comprising rasagiline mesylate, comprising:
step 1: preparing a drug-containing matrix layer, wherein 0.5-5.0% of rasagiline mesylate, 0.1-5.0% of a release promoter, 1-15% of a penetration enhancer, 0.5-10% of a plasticizer, and 0.001-0.5% of an antioxidant are weighed, dissolved in a solvent, and mixed and stirred until all solids are completely dissolved to provide a homogeneous solution; 3-25% of crospovidone is added into the homogeneous solution, followed by pre-mixing and swelling sufficiently and then homogenizing, to provide a white emulsion liquid; and 40-80% of a binder is added to the white emulsion liquid, followed by mixing and stirring uniformly to provide an intermediate solution;
step 2: coating and drying to remove the solvent, wherein the intermediate solution is coated on a protective layer, with a wet film thickness of 100-500 µm, and then dried to provide a dried matrix, with a dried film thickness of 20-250 µm; and
step 3: laminating, compounding and cutting, wherein the backing layer is laminated and compounded with the dried matrix, and then cut into patches,
wherein the release promoter is selected from one or a combination of several of levulinic acid, octanoic acid, and undecanoic acid, a mass ratio of rasagiline mesylate to crospovidone is 1:5 to 1:15, and a content of the binder is finally adjusted to formulate a mass percentage of 100%.

22. The preparation method according to claim 20 or 21, wherein the solvent in step 1 is selected from one or a combination of two of anhydrous ethanol and isopropyl alcohol.

23. The preparation method according to claim 22, wherein the solvent in step 1 is selected from anhydrous ethanol.

24. The preparation method according to claim 21 or 22, wherein drying in step 2 is carried out by a three-stage drying method, with drying at 20-30 °C for 2-10 min in a first stage, drying at 45-65 °C for 2-10 min in a second stage, and drying at 65-85 °C for 2-10 min in a third stage.

25. The preparation method according to claim 24, wherein the first stage of the drying in step 2 is drying at 20-25 °C for 2-10 min.

26. A matrix mixture, for preparing a transdermal patch comprising rasagiline mesylate, comprising following components in mass percentage: 0.5-5.0% of rasagiline mesylate, 40-80% of a binder, 3-25% of crospovidone, 1-5% of a crystallization inhibitor, 1-15% of a penetration enhancer, 0.1-5.0% of a release promoter, 1-10% of a plasticizer, and 0.001-0.5% of an antioxidant, with a mass ratio of rasagiline mesylate to crospovidone being 1:5 to 1:15, and a content of the binder being finally adjusted to formulate a mass percentage of 100%, wherein the release promoter is selected from one or a combination of several of levulinic acid, octanoic acid, and undecanoic acid.

27. A matrix mixture, for preparing a transdermal patch comprising rasagiline mesylate, comprising following components in mass percentage: 0.5-5.0% of rasagiline mesylate, 40-80% of a binder, 3-25% of crospovidone, 0.1-5.0% of a release promoter, 1-15% of a penetration enhancer, 0.5-10% of a plasticizer, and 0.001-0.5% of an antioxidant, with a mass ratio of rasagiline mesylate to crospovidone being 1:5 to 1:15, and a content of the binder being finally adjusted to formulate a mass percentage of 100%, wherein the release promoter is selected from one or a combination of several of levulinic acid, octanoic acid, and undecanoic acid.

28. The matrix mixture according to claim 26 or 27, further comprising anhydrous ethanol or isopropyl alcohol.
